# EUROPÄISCHE PATENTSCHRIFT

(11) **EP 4 196 082 B1**
(45) Veröffentlichungstag und Bekanntmachung des Hinweises auf die Patenterteilung: **31.12.2025**
(21) Anmeldenummer: 21745261.4
(22) Anmeldetag: 07.07.2021
(51) Int. Cl.: A61K 8/06, A61K 8/37, A61K 8/39, A61K 8/55, A61K 8/92, A61Q 19/00

(54) **KOSMETISCHE ETHANOL-IN-ÖL-EMULSION**
COSMETIC ETHANOL-IN-OIL EMULSION
ÉMULSION D'ÉTHANOL-DANS-HUILE COSMÉTIQUE

(30) Priorität: 13.08.2020 DE 102020210312
(43) Veröffentlichungstag der Anmeldung: 21.06.2023
(73) Patentinhaber: Beiersdorf AG, 22529 Hamburg (DE)
(72) Erfinder: SIVACILAR, Berkay Daniel, 20539 Hamburg (DE); GOULET-HANSSENS, Alexis, 20255 Hamburg (DE); VON WEDEL-PARLOW, Magdalena, 22527 Hamburg (DE)
(74) Vertreter: Beiersdorf AG
(86) Internationale Anmeldenummer: PCT/EP2021/068817
(87) Internationale Veröffentlichungsnummer: WO 2022/033774

(56) Entgegenhaltungen:
- US-A1- 2010 015 070
- US-A1- 2013 085 186
- US-A1- 2017 216 205
- US-B2- 10 603 256

## Beschreibung

Die vorliegende Erfindung betrifft eine kosmetische Emulsion aus Ethanol, einer Ölphase, einem Emulgator und gegebenenfalls weiteren kosmetischen Wirk-Hilfs und Zusatzstoffen, welche im Ethanol oder der Ölphase gelöst vorliegen sowie die Verwendung derartiger Emulsionen als kosmetische Zubereitung.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte (sowie einige Hautreinigungsprodukte) bestehen in der Regel aus Emulsionen. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Neben Emulsionen, die aus einer Wasserphase und einer Ölphase gebildet werden, sind grundsätzlich auch andere Systeme von mit Ölen nicht mischbaren flüssigen Phasen denkbar, die in der Kosmetik bisher jedoch -nicht zuletzt wegen formulierungstechnischer Schwierigkeitenkeine Rolle spielen.

Es war daher die Aufgabe der vorliegenden Erfindung, ein alternatives Emulsionssystem für die Kosmetik zu entwickeln, dass Lager- und Temperatur-stabil ist und eine angenehme Sensorik aufweist. Insbesondere Phasentrennungen im Verlaufe der Lagerung sollten vermieden werden. Darüber hinaus sollten sich kosmetische Wirkstoffe, wie beispielsweise UV-Filter, Antioxidantien, Haut-befeuchtende Stoffe, Antifaltenwirkstoffe, Parfümstoffe etc. stabil und in wirksamer Menge einarbeiten lassen.

Nachteilig an kosmetischen Emulsionen des Standes der Technik ist darüber hinaus der Umstand, dass der Gehalt an Wasser dazu führt, dass die Zubereitung nicht mehr mikrobiell stabil ist und das Kosmetikum zur Haltbarmachung mit Konservierungsmitteln versetzt werden muss. An sich ist der Einsatz von Konservierungsmitteln in Kosmetika unkritisch, doch wird von unterschiedlicher Seite immer wieder deren Verträglichkeit in Frage gestellt. Ob und in welchem Umfang derlei Bedenken einer wissenschaftlichen Überprüfung Stand halten, kann im Rahmen der vorliegenden Offenbarung dahingestellt bleiben. Tatsache ist jedoch, dass ein nicht unbedeutender Teil der Verbraucher "Konservierungsmittel-freie" Produkte wünscht.

Es war daher die Aufgabe der vorliegenden Erfindung, eine kosmetische Emulsion zu entwickeln, die frei ist von Konservierungsstoffen. Dabei werden erfindungsgemäß unter Konservierungsstoffen diejenigen Verbindungen verstanden, die zum Prioritätszeitpunkt dieser Anmeldung in der Positiv-Liste der Kosmetikrichtlinie der Europäischen Union aufgelistet in Anhang VI aufgelistet sind.

Aufgrund ihres guten Emulgier-Vermögens stellen die Polyethylenglycol- und Polypropylenglycol-Ether und -Ester eine viel verwendete Gruppe von kosmetischen Emulgatoren dar. Nachteilig am Stande der Technik ist jedoch der Umstand, dass diese Emulgatoren im Verdacht stehen, die Hautpenetration von Stoffen begünstigen, die nicht in die tieferen Hautschichten eindringen sollen. Ob und in welchem Umfang diese Eigenschaft ein tatsächliches Problem für die Anwender darstellt, kann im Rahmen der vorliegenden Offenbarung ebenfalls dahingestellt bleiben. Tatsache ist auch hier, dass der Einsatz von Polyethylenglycol bzw. Polypropylenglycol -ethern und - estern bei den Verbrauchern zunehmend unerwünscht ist und es die Aufgabe der vorliegenden Erfindung war, auf diese weit verbreiteten Emulgator-Systeme zu verzichten.

Üblicherweise werden die wässrigen Phasen einer kosmetischen Emulsion mit Trinkwasser hergestellt. Aufgrund der unterschiedlichen Gehalte an Alkali- und Erdalkalisalzen kommt es bei der Herstellung der wässrigen Phasen immer wieder zu Problemen, da die Salzgehalte die Stabilität der Emulsion beeinflussen. Um an unterschiedlichen Herstellungsorten (mit unterschiedlichen Wasserqualitäten) die gleiche kosmetische Rezeptur herstellen zu können, muss das eingesetzte Trinkwasser in der Regel erst einmal einem aufwendigen Aufbereitungsprozess (z. B. Ionenaustausch) unterworfen werden, um vergleichbare Wasserqualitäten zu erzeugen mit denen dann die Emulsion hergestellt werden kann.

Es war daher die Aufgabe der vorliegenden Erfindung, eine kosmetische Emulsion zu entwickeln, die ohne den Einsatz von Trinkwasser in der wässrigen Phase auskommt.

Überraschend gelöst werden die Aufgaben durch eine kosmetische Ethanol-in Öl-Emulsion gemäß Anspruch 1.

Überraschend gelöst werden die Aufgaben ferner durch die Verwendung einer Ethanol-in Öl-Emulsion gemäß Anspruch 2.

Überraschend gelöst werden die Aufgaben nicht zuletzt durch ein Verfahren zur Herstellung einer Ethanol-in Öl-Emulsion gemäß Anspruch 3.

Zwar kennt der Fachmann die US 2013/0085186 A1, US 2010/0015070 A1, US 2017/0216205 A1 und US 10,603,256 B2, doch konnten diese Schriften nicht den Weg zur vorliegenden Erfindung weisen.

Im Rahmen der vorliegenden Offenbarung beziehen sich die Formulierungen "erfindungsgemäß", "erfindungsgemäße Zubereitung" etc. immer auf die erfindungsgemäßen Zubereitungen, Verfahren und Verwendungen, d.h. auch auf Zubereitungen, in denen die erfindungsgemäßen Verwendungen verwirklicht werden sowie Zubereitungen, mit denen das erfindungsgemäße Verfahren verwirklicht wird.

Es ist erfindungsgemäß vorteilhaft, wenn als Emulgator ein oder mehrere Polyglycerylester oder Sojalecithin eingesetzt wird.

Es ist erfindungsgemäß vorteilhaft, wenn der Ethanolgehalt der Zubereitung 5 bis 25 Gewichts-%, bezogen auf das Gesamtgewicht der Emulsion, beträgt. Dabei ist ein Ethanolgehalt von 8 bis 12 Gewichts-%, bezogen auf das Gesamtgewicht der Zubereitung, erfindungsgemäß bevorzugt. Besonders gute Ergebnisse wurden mit einem Ethanolgehalt von 10 Gewichts-%, bezogen auf das Gesamtgewicht der Zubereitung, erzielt.

Vorteilhafte Ausführungsformen der vorliegenden Erfindung sind dadurch gekennzeichnet, dass die Ölphase eine Oberflächenspannung von 29-35 mN/m aufweist.

Dabei ist es erfindungsgemäß bevorzugt, wenn die Ölphase aus einem oder mehreren Ölen mit einer Oberflächenspannung von 29-35 mN/m gebildet wird.

Erfindungsgemäß vorteilhaft ist es, wenn die Ölphase ein oder mehrere Öle gewählt aus der Gruppe der Verbindungen Sonnenblumenkernöl (INCI: Sunflower Seed Oil), Kokosglyceriden (INCI: Coco Glycerides), Mandelöl (INCI: Almond Oil), Rizinusöl (INCI: Castor Seed Oil), Jojobaöl (INCI: Jojoba Seed Oil), Capryl-/Caprinsäuretriglyceriden (INCI: Caprylic/Capric Triglycerides), Avocadoöl (INCI: Avocado Oil), Olivenöl (INCI: Olive Oil), hydriertes Rapsöl (INCI: Hydrogenated Rapseed Oil) enthält.

Darüber hinaus ist es vorteilhaft im Sinne der vorliegenden Erfindung, wenn die Ölphase ein oder mehrere Öle gewählt aus der Gruppe der Verbindungen und Ethylhexyl-2-cyano-3,3-diphenylacrylat (INCI: Octocrylen), 2-Ethylhexyl 2-hydroxybenzoat (INCI: Ethylhexyl Salicylate) und 3,3,5-Trimethylcyclohexyl 2-hydroxybenzoat (INCI: Homosalate) enthält.

Insbesondere ist es erfindungsgemäß bevorzugt, wenn die Ölphase 2-Ethylhexyl 2-hydroxybenzoat (INCI: Ethylhexyl Salicylate) in einer Konzentration von 3 bis 5 Gewichts-%, bezogen auf das Gesamtgewicht der Zubereitung, enthält.

Im Hinblick auf den Einsatz von Ethylhexyl-2-cyano-3,3-diphenylacrylat (INCI: Octocrylen) ist festzuhalten, das erfindungsgemäß 2 Alternativen denkbar sind: Ein Gehalt von Octocrylen und der Verzicht auf Octocrylen. Erfindungsgemäß bevorzugt ist dabei die Variante ohne Octocrylen.

Vorteilhaft im Sinne der vorliegenden Erfindung ist es, wenn die erfindungsgemäße Zubereitung Glycerin enthält.

In einem solchen Falle ist es erfindungsgemäß bevorzugt, wenn die Zubereitung Glycerin in einer Konzentration von 1,0 bis 7,0 Gewichts-%, bezogen auf das Gesamtgewicht der Zubereitung, enthält.

Die erfindungsgemäße Emulsion kann darüber hinaus UV-Filter enthalten. Diese können beispielsweise gewählt werden aus der Gruppe der Verbindungen 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-Benzylidencampher; 4-(Dimethylamino)-benzoesäure(2-ethylhexyl)ester; 4-(Dimethylamino)benzoesäureamylester; 4-Methoxybenzalmalonsäuredi(2-ethylhexyl)ester; 4- 4-Methoxyzimtsäureisoamylester; 2-Hydroxy-4-methoxy-4'-methylbenzophenon; 2,2'-Dihydroxy-4-methoxybenzophenon; 4-(tert.-Butyl)-4'-methoxydibenzoylmethan; Homomenthylsalicylat; 2-Ethylhexyl-2-hydroxybenzoat; Dimethicodiethylbenzalmalonat; 3-(4-(2,2-bis Ethoxycarbonylvinyl)-phe-noxy)propenyl)-methoxysiloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'- Di-neopentylaminobenzalmalonat)-6-(4"-butylaminobenzoat)-s-triazin, Titandioxid, Zinkoxid und den Merocyaninen.

Erfindungsgemäß bevorzugt ist es dabei, wenn die Zubereitung einen oder mehrere UV-Filter gewählt aus der Gruppe der Verbindungen 4-(tert.-Butyl)-4'-methoxydibenzoylmethan, Hexyl 2-[4-(diethylamino)-2-hydroxybenzoyl]benzoate (INCI: Diethylamino hydroxybenzoyl hexyl benzoate), 2,4,6-Tris-[anilino-(p-carbo-2'-ethyl-1'-hexyloxy)]-1,3,5-triazin (INCI: Ethylhexyl Triazone), 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine),enthält.

Die erfindungsgemäße Emulsion kennt insbesondere zwei erfindungsgemäß vorteilhafte Emulgator-Systeme: Zum einen können Polyglycerylester eingesetzt werden, zum anderen Sojalecithine.

Werden Polyglycerylester als Emulgator eingesetzt, so sind diese erfindungsgemäß vorteilhaft dadurch gekennzeichnet, dass als Emulgator ein Polyglyceryl-10 Fettsäureester eingesetzt wird.

Dabei ist es erfindungsgemäß bevorzugt, wenn Polyglyceryl-10 Fettsäureester in einer Konzentration von 0,1 bis 2,0 Gewichts-%, bezogen auf das Gesamtgewicht der Zubereitung, eingesetzt werden.

Als erfindungsgemäß bevorzugte Polyglyceryl-10 Fettsäureester werden dabei Polyglyceryl-10 Stearat (INCI Polyglyceryl-10 Stearate) und/oder Polyglyceryl-10 Oleat (INCI: Polyglyceryl-10 Oleate) eingesetzt.

Erfindungsgemäß besonders bevorzugt ist dabei der Einsatz von oder Polyglyceryl-10 Oleat (INCI: Polyglyceryl-10 Oleate).

Werden hingegen Sojalecithine als Emulgatoren eingesetzt, so ist es erfindungsgemäß vorteilhaft, wenn Sojalecithin in einer Konzentration von 0,1 bis 2,0 Gewichts-%, bezogen auf das Gesamtgewicht der Zubereitung, eingesetzt wird.

Erfindungsgemäß bevorzugt ist es, wenn die Zubereitung frei ist von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), 2-Ethylhexyl-2-cyano-3,3-diphenylacrylat, Homomenthylsalicylat, Parabenen (insbesondere Propyl- und Butylparaben), Methylisothiazolinon, Chlormethylisothiazolinon und DMDM-Hydantoin, Polyethylenglycolethern oder Polyethylenglycolestern, sowie frei ist von Mineralölen, Silikonölen und Mineralwachsen.

Erfindungsgemäß vorteilhafte Ausführungsformen der vorliegenden Erfindung sind nicht zuletzt dadurch gekennzeichnet, dass die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherol, Tocopherylacetat, Dihydroxyaceton; Polydocanol, Thiamidol, Glycerylglycose, Hyaluronsäure und/oder deren Salze und/oder Licochalcon A enthält.

Im Hinblick auf die Viskosität ist es erfindungsgemäß von Vorteil, wenn die erfindungsgemäße Emulsion eine Viskosität von weniger als 1500 mPas (gemessen mit *Rheomat R123* von "proRheo" bei 25°C, bei konstanter Drehzahl für 30 Sekunden) aufweist.

Das erfindungsgemäße Verfahren ist erfindungsgemäß vorteilhaft dadurch gekennzeichnet, dass die Homogenisierung mit Hilfe eines Ultra-Turrax durchgeführt wird. Hierfür kommt beispielsweise das Model T-25 in Frage. Üblicherweise erfolgt diese Homogenisierung dann bei 11000 U/min. während einer Dauer von etwa 5 Minuten.

Enthält die Ölphase lipophile Feststoffe, beispielsweise UV-Filter, so werden diese Komponenten gegebenenfalls unter Erwärmen des Öls unter Rühren eingearbeitet, bevor de Ölphase dann mit der Ethanolphase vereinigt wird.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| INCI | Bsp. 1 | Bsp. 2 | Bsp. 3 | Bsp. 4 | Bsp. 5 | Bsp. 6 | Bsp. 7 | Bsp. 8 | Bsp. 9 | Bsp. 10 | Bsp. 11 |
|---|---|---|---|---|---|---|---|---|---|---|---|
| Alcohol Denat. + Aqua | 20,0 0 | 20,0 0 | 10,0 0 | 10,0 0 | 20,0 0 | 20,0 0 | 10,0 0 | 10,0 0 | 9,95 | 10,00 | 10,00 |
| Polyglyceryl-10 Oleate | 0,10 | 1,00 | 0,10 | 1,00 | 0,10 | 1,00 | 0,10 | 1,00 | | | |
| Lecithin | | | | | | | | | 0,99 5 | | |
| Polyglyceryl-10 Stearate | | | | | | | | | | 1,00 | |
| Polyglyceryl-10 Pentaisosteara te | | | | | | | | | | | 1,00 |
| Helianthus Annuus(Sunflo wer) Seed Oil | 79,9 0 | 79,0 0 | 89,9 0 | 89,0 0 | | | | | 89,0 5 | 89,00 | 89,00 |
| Olus (Vegetable) Oil | | | | | 79,9 0 | 79,0 0 | 89,9 0 | 89,0 0 | | | |

| INCI | Bsp. 12 | Bsp. 13 | Bsp. 14 | Bsp. 15 | Bsp. 16 | Bsp. 17 | Bsp. 18 | Bsp. 19 | Bsp. 20 | Bsp. 21 |
|---|---|---|---|---|---|---|---|---|---|---|
| Alcohol Denat. + Aqua | 10,00 | 10,00 | 10,00 | 10,00 | 10,00 | 10,00 | 12,17 | 12,15 | 10,26 | 12,1 7 |
| Polyglyceryl-10 Oleate | 1,00 | 1,00 | 1,00 | 1,00 | 1,00 | 1,00 | 0,87 | 1,00 | 0,77 | 0,86 |
| Helianthus Annuus(Sunflo wer) Seed Oil | | | | | | | | 73,82 | 58,98 | |
| Cocoglyceride s | 89,00 | | | | | | 73,90 | | | |
| Prunus Amygdalus Dulcis (Sweet Almond) Oil | | 89,00 | | | | | | | | |
| Ricinus Communis Seed (Castor) Oil | | | 84,00 | | | | | | | |
| Persea Gratissima (Avocado) Oil | | | | 89,00 | | | | | | |
| Olea Europaea Fruit (Olive) Oil | | | | | 89,00 | | | | | 73,9 1 |
| Caprylic/Capri c Triglycerides | | | | | | 89,00 | | | | |
| Glycerin | | | 5,00 | | | | | | | |
| Butyl Methoxydiben zoylmethane | | | | | | | 4,35 | 4,34 | 5,12 | 4,34 |
| Octocrylene | | | | | | | | | 23,08 | |
| Ethylhexyl Triazone | | | | | | | 4,35 | 4,34 | | 4,34 |
| Bis-Ethylhexyloxyp henol | | | | | | | 4,35 | 4,34 | | 4,34 |
| Methoxypheny l Triazone | | | | | | | | | | |

## Patentansprüche

1. Kosmetische Ethanol-in Öl-Emulsion aus
a) Ethanol
b) einer Ölphase
c) einem Emulgator
d) gegebenenfalls weiteren kosmetischen Wirk-Hilfs und Zusatzstoffen, welche im Ethanol oder der Ölphase gelöst vorliegen, **dadurch gekennzeichnet, dass** der Wassergehalt der Zubereitung weniger als 5 Gewichts-%, bezogen auf das Gesamtgewicht der Emulsion, beträgt

2. Verwendung einer Ethanol-in-Öl-Emulsion aus
a) Ethanol
b) einer Ölphase
c) einem Emulgator
d) gegebenenfalls weiteren kosmetischen Wirk-Hilfs und Zusatzstoffen, welche im Ethanol oder der Ölphase gelöst vorliegen, **dadurch gekennzeichnet, dass** der Wassergehalt der Zubereitung weniger als 5 Gewichts-%, bezogen auf das Gesamtgewicht der Emulsion, beträgt, als Kosmetikum.

3. Verfahren zur Herstellung einer Ethanol-in-Öl-Emulsion, bei dem in einem ersten Schritt der Emulgator im Ethanol gelöst wird, in einem zweiten Schritt die Ölphase zur Ethanol/Emulgatormischung unter Rühren zugesetzt wird und anschließend homogenisiert wird, **dadurch gekennzeichnet, dass** der Wassergehalt der Zubereitung weniger als 5 Gewichts-%, bezogen auf das Gesamtgewicht der Emulsion, beträgt.

4. Emulsion nach Anspruch 1, Verwendung nach Anspruch 2 oder Verfahren nach Anspruch 3, **dadurch gekennzeichnet, dass** als Emulgator ein oder mehrere Polyglycerylester oder Sojalecithin eingesetzt werden.

5. Emulsion, Verwendung oder Verfahren nach einem der vorhergehenden Ansprüche, **dadurch gekennzeichnet, dass** der Ethanolgehalt der Zubereitung 5 bis 25 Gewichts%, bezogen auf das Gesamtgewicht der Emulsion, beträgt.

6. Emulsion, Verwendung oder Verfahren nach einem der vorhergehenden Ansprüche, **dadurch gekennzeichnet, dass** die Ölphase eine Oberflächenspannung von 29-35 mN/m aufweist.

7. Emulsion, Verwendung oder Verfahren nach einem der vorhergehenden Ansprüche, **dadurch gekennzeichnet, dass** die Ölphase aus einem oder mehreren Ölen mit einer Oberflächenspannung von 29-35 mN/m gebildet wird.

8. Emulsion, Verwendung oder Verfahren nach einem der vorhergehenden Ansprüche, **dadurch gekennzeichnet, dass** die Ölphase ein oder mehrere Öle gewählt aus der Gruppe der Verbindungen Sonnenblumenkernöl (INCI: Sunflower Seed Oil), Kokosglyceriden (INCI: Coco Glycerides), Mandelöl (INCI: Almond Oil), Rizinusöl (INCI: Castor Seed Oil), Jojobaöl (INCI: Jojoba Seed Oil), Capryl-/Caprinsäuretriglyceriden (INCI: Caprylic/Capric Triglycerides), Avocadoöl (INCI: Avocado Oil), Olivenöl (INCI: Olive Oil), hydriertes Rapsöl (INCI: Hydrogenated Rapseed Oil) enthält.

9. Emulsion, Verwendung oder Verfahren nach einem der vorhergehenden Ansprüche, **dadurch gekennzeichnet, dass** die Ölphase ein oder mehrere Öle gewählt aus der Gruppe der Verbindungen und Ethylhexyl-2-cyano-3,3-diphenylacrylat (INCI: Octocrylen), 2-Ethylhexyl 2-hydroxybenzoat (INCI: Ethylhexyl Salicylate) und 3,3,5-Trimethylcyclohexyl 2-hydroxybenzoat (INCI: Homosalate) enthält.

10. Emulsion, Verwendung oder Verfahren nach einem der vorhergehenden Ansprüche, **dadurch gekennzeichnet, dass** die Ölphase 2-Ethylhexyl 2-hydroxybenzoat (INCI: Ethylhexyl Salicylate) in einer Konzentration von 3 bis 5 Gewichts-%, bezogen auf das Gesamtgewicht der Zubereitung, enthält.

11. Emulsion, Verwendung oder Verfahren nach einem der vorhergehenden Ansprüche, **dadurch gekennzeichnet, dass** die Zubereitung Glycerin enthält.

12. Emulsion, Verwendung oder Verfahren nach einem der vorhergehenden Ansprüche, **dadurch gekennzeichnet, dass** die Zubereitung Glycerin in einer Konzentration von 1,0 bis 7,0 Gewichts-%, bezogen auf das Gesamtgewicht der Zubereitung, enthält.

13. Emulsion, Verwendung oder Verfahren nach einem der vorhergehenden Ansprüche, **dadurch gekennzeichnet, dass** die Zubereitung einen oder mehrere UV-Filter gewählt aus der Gruppe der Verbindungen 4-(tert.-Butyl)-4'-methoxydibenzoylmethan, Hexyl 2-[4-(diethylamino)-2-hydroxybenzoyl]benzoate (INCI: Diethylamino hydroxybenzoyl hexyl benzoate), 2,4,6-Tris-[anilino-(p-carbo-2'-ethyl-1'-hexyloxy)]-1,3,5-triazin (INCI: Ethylhexyl Triazone), 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine),enthält.

14. Emulsion, Verwendung oder Verfahren nach einem der vorhergehenden Ansprüche, **dadurch gekennzeichnet, dass** als Emulgator ein Polyglyceryl-10 Fettsäureester eingesetzt wird.

15. Emulsion, Verwendung oder Verfahren nach einem der vorhergehenden Ansprüche, **dadurch gekennzeichnet, dass** als Emulgator ein Polyglyceryl-10 Fettsäureester in einer Konzentration von 0,1 bis 2,0 Gewichts-%, bezogen auf das Gesamtgewicht der Zubereitung, eingesetzt wird.

16. Emulsion, Verwendung oder Verfahren nach einem der vorhergehenden Ansprüche, **dadurch gekennzeichnet, dass** als Emulgator Polyglyceryl-10 Stearat (INCI Polyglyceryl-10 Stearate) und/oder Polyglyceryl-10 Oleat (INCI: Polyglyceryl-10 Oleate) eingesetzt werden.

17. Emulsion, Verwendung oder Verfahren nach einem der vorhergehenden Ansprüche, **dadurch gekennzeichnet, dass** als Emulgator Sojalecithin in einer Konzentration von 0,1 bis 2,0 Gewichts-%, bezogen auf das Gesamtgewicht der Zubereitung, eingesetzt wird.

18. Emulsion, Verwendung oder Verfahren nach einem der vorhergehenden Ansprüche, **dadurch gekennzeichnet, dass** die Zubereitung frei ist von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), 2-Ethylhexyl-2-cyano-3,3-diphenylacrylat, Homomenthylsalicylat, Parabenen (insbesondere Propylund Butylparaben), Methylisothiazolinon, Chlormethyl-isothiazolinon und DMDM-Hydantoin, Polyethylenglycolethern oder Polyethylenglycolestern, sowie frei ist von Mineralölen, Silikonölen und Mineralswachsen.

19. Emulsion, Verwendung oder Verfahren nach einem der vorhergehenden Ansprüche, **dadurch gekennzeichnet, dass** die Zubereitung eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, natürliche und/oder synthetische Isoflavonoide, Flavonoide, Kreatin, Kreatinin, Taurin, β-Alanin, Panthenol, Magnolol, Honokiol, Tocopherol, Tocopherylacetat, Dihydroxyaceton; Polydocanol, Thiamidol, Glycerylglycose, Hyaluronsäure und/oder deren Salze und/oder Licochalcon A enthält.

## Claims

1. Cosmetic ethanol-in-oil emulsion of
a) ethanol
b) an oil phase
c) an emulsifier
d) optionally further cosmetic active ingredients, auxiliaries and additives which are dissolved in the ethanol or the oil phase, **characterized in that** the water content of the preparation is less than 5% by weight, based on the total weight of the emulsion.

2. Use of an ethanol-in-oil emulsion of
a) ethanol
b) an oil phase
c) an emulsifier
d) optionally further cosmetic active ingredients, auxiliaries and additives which are dissolved in the ethanol or the oil phase, **characterized in that** the water content of the preparation is less than 5% by weight, based on the total weight of the emulsion, as a cosmetic.

3. Process for producing an ethanol-in-oil emulsion, in which the emulsifier is dissolved in the ethanol in a first step, the oil phase is added to the ethanol/emulsifier mixture with stirring in a second step and then homogenization is performed, **characterized in that** the water content of the preparation is less than 5% by weight, based on the total weight of the emulsion.

4. Emulsion according to Claim 1, use according to Claim 2 or process according to Claim 3, **characterized in that** one or more polyglyceryl esters or soya lecithin are used as emulsifier.

5. Emulsion, use or process according to any of the preceding claims, **characterized in that** the ethanol content of the preparation is 5% to 25% by weight, based on the total weight of the emulsion.

6. Emulsion, use or process according to any of the preceding claims, **characterized in that** the oil phase has a surface tension of 29-35 mN/m.

7. Emulsion, use or process according to any of the preceding claims, **characterized in that** the oil phase is formed from one or more oils having a surface tension of 29-35 mN/m.

8. Emulsion, use or process according to any of the preceding claims, **characterized in that** the oil phase comprises one or more oils selected from the group of compounds sunflower seed oil (INCI: Sunflower Seed Oil), cocoglycerides (INCI: Cocoglycerides), almond oil (INCI: Almond Oil), castor oil (INCI: Castor Seed Oil), jojoba oil (INCI: Jojoba Seed Oil), caprylic/capric triglycerides (INCI: Caprylic/Capric Triglycerides), avocado oil (INCI: Avocado Oil), olive oil (INCI: Olive Oil), hydrogenated rapeseed oil (INCI: Hydrogenated Rapeseed Oil).

9. Emulsion, use or process according to any of the preceding claims, **characterized in that** the oil phase comprises one or more oils selected from the group of compounds and ethylhexyl 2-cyano-3,3-diphenylacrylate (INCI: Octocrylene), 2-ethylhexyl 2-hydroxybenzoate (INCI: Ethylhexyl Salicylate) and 3,3,5-trimethylcyclohexyl 2-hydroxybenzoate (INCI: Homosalate).

10. Emulsion, use or process according to any of the preceding claims, **characterized in that** the oil phase comprises 2-ethylhexyl 2-hydroxybenzoate (INCI: Ethylhexyl Salicylate) at a concentration of 3% to 5% by weight, based on the total weight of the preparation.

11. Emulsion, use or process according to any of the preceding claims, **characterized in that** the preparation comprises glycerin.

12. Emulsion, use or process according to any of the preceding claims, **characterized in that** the preparation comprises glycerin at a concentration of 1.0% to 7.0% by weight, based on the total weight of the preparation.

13. Emulsion, use or process according to any of the preceding claims, **characterized in that** the preparation comprises one or more UV filters selected from the group of compounds 4-(tert-butyl)-4'-methoxydibenzoylmethane, hexyl 2-[4-(diethylamino)-2-hydroxybenzoyl]benzoate (INCI: Diethylamino Hydroxybenzoyl Hexyl Benzoate), 2,4,6-tris[anilino-(p-carbo-2'-ethyl-1'-hexyloxy)]-1,3,5-triazine (INCI: Ethylhexyl Triazone), 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine).

14. Emulsion, use or process according to any of the preceding claims, **characterized in that** a polyglyceryl-10 fatty acid ester is used as emulsifier.

15. Emulsion, use or process according to any of the preceding claims, **characterized in that** a polyglyceryl-10 fatty acid ester is used as emulsifier at a concentration of 0.1% to 2.0% by weight, based on the total weight of the preparation.

16. Emulsion, use or process according to any of the preceding claims, **characterized in that** polyglyceryl-10 stearate (INCI: Polyglyceryl-10 Stearate) and/or polyglyceryl-10 oleate (INCI: Polyglyceryl-10 Oleate) are used as emulsifier.

17. Emulsion, use or process according to any of the preceding claims, **characterized in that** soya lecithin is used as emulsifier at a concentration of 0.1% to 2.0% by weight, based on the total weight of the preparation.

18. Emulsion, use or process according to any of the preceding claims, **characterized in that** the preparation is free of 3-(4-methylbenzylidene)camphor, 2-hydroxy-4-methoxybenzophenone (INCI: Oxybenzone), 2-ethylhexyl 4-methoxycinnamate (INCI: Octyl Methoxycinnamate), 2-ethylhexyl 2-cyano-3,3-diphenylacrylate, homomenthyl salicylate, parabens (particularly propyl and butyl paraben), methylisothiazolinone, chloromethylisothiazolinone and DMDM hydantoin, polyethylene glycol ethers or polyethylene glycol esters, and is free of mineral oils, silicone oils and mineral waxes.

19. Emulsion, use or process according to any of the preceding claims, **characterized in that** the preparation comprises one or more compounds selected from the group of compounds alpha-lipoic acid, folic acid, phytoene, D-biotin, coenzyme Q10, alpha-glucosylrutin, carnitine, carnosine, natural and/or synthetic isoflavonoids, flavonoids, creatine, creatinine, taurine, β-alanine, panthenol, magnolol, honokiol, tocopherol, tocopheryl acetate, dihydroxyacetone; polydocanol, Thiamidol, glycerylglucose, hyaluronic acid and/or salts thereof and/or licochalcone A.

## Revendications

1. Émulsion cosmétique éthanol-dans-eau constituée par
a) de l'éthanol,
b) une phase huileuse,
c) un émulsifiant,
d) le cas échéant d'autres adjuvants actifs et additifs, qui se trouvent sous forme dissoute dans l'éthanol ou dans la phase huileuse, **caractérisée en ce que** la teneur en eau de la préparation est inférieure à 5% en poids, par rapport au poids total de l'émulsion.

2. Utilisation d'une émulsion éthanol-dans-eau constituée par
a) de l'éthanol,
b) une phase huileuse,
c) un émulsifiant,
d) le cas échéant d'autres adjuvants actifs et additifs, qui se trouvent sous forme dissoute dans l'éthanol ou dans la phase huileuse, **caractérisée en ce que** la teneur en eau de la préparation est inférieure à 5% en poids, par rapport au poids total de l'émulsion, en tant que cosmétique.

3. Procédé de préparation d'une émulsion éthanol-dans-eau, dans lequel, dans une première étape, l'émulsifiant est dissous dans de l'éthanol, dans une deuxième étape, la phase huileuse est ajoutée au mélange éthanol/émulsifiant et ensuite on homogénéise, **caractérisé en ce que** la teneur en eau de la préparation est inférieure à 5% en poids, par rapport au poids total de l'émulsion.

4. Émulsion selon la revendication 1, utilisation selon la revendication 2 ou procédé selon la revendication 3, caractérisé(e) en ce qu'on utilise, comme émulsifiant, un ou plusieurs esters de polyglycéryle ou de la lécithine de soja.

5. Émulsion, utilisation ou procédé selon l'une des revendications précédentes, caractérisé(e) en ce que la teneur en éthanol de la préparation est de 5 à 25% en poids, par rapport au poids total de l'émulsion.

6. Émulsion, utilisation ou procédé selon l'une des revendications précédentes, caractérisé(e) en ce que la phase huileuse présente une tension superficielle de 29-35 mN/m.

7. Émulsion, utilisation ou procédé selon l'une des revendications précédentes, caractérisé(e) en ce que la phase huileuse est formée à partir d'une ou de plusieurs huiles présentant une tension superficielle de 29-35 mN/m.

8. Émulsion, utilisation ou procédé selon l'une des revendications précédentes, caractérisé(e) en ce que la phase huileuse contient une ou plusieurs huiles choisies dans le groupe des composés huile de graines de tournesol (INCI : Sunflower Seed Oil), glycérides de coco (INCI : Coco Glycerides), huile d'amande (INCI : Almond Oil), huile de ricin (INCI : Castor Seed Oil), huile de jojoba (INCI : Jojoba Seed Oil), triglycérides de l'acide caprylique/caprique (INCI : Caprylic/Capric Triglycerides), huile d'avocat (INCI : Avocado Oil), huile d'olive (INCI : Olive Oil), huile de colza hydrogénée (INCI : Hydrogenated Rapseed Oil).

9. Émulsion, utilisation ou procédé selon l'une des revendications précédentes, caractérisé(e) en ce que la phase huileuse contient une ou plusieurs huiles choisies dans le groupe des composés et 2-cyano-3,3-diphénylacrylate d'éthylhexyle (INCI : Octocrylene), 2-hydroxybenzoate de 2-éthylhexyle (INCI : Ethylhexyl Salicylate) et 2-hydroxybenzoate de 3,3,5-triméthylcyclohexyle (INCI : Homosalate).

10. Émulsion, utilisation ou procédé selon l'une des revendications précédentes, caractérisé(e) en ce que la phase huileuse contient du 2-hydroxybenzoate de 2-éthylhexyle (INCI : Ethylhexyl Salicylate) en une concentration de 3 à 5% en poids, par rapport au poids total de la préparation.

11. Émulsion, utilisation ou procédé selon l'une des revendications précédentes, caractérisé(e) en ce que la préparation contient du glycérol.

12. Émulsion, utilisation ou procédé selon l'une des revendications précédentes, caractérisé(e) en ce que la préparation contient du glycérol en une concentration de 1,0 à 7,0% en poids par rapport au poids total de la préparation.

13. Émulsion, utilisation ou procédé selon l'une des revendications précédentes, caractérisé(e) en ce que la préparation contient un ou plusieurs filtres UV, choisis dans le groupe des composés 4-(tert-butyl)-4'-méthoxydibenzoylméthane, 2-[4-(diéthylamino)-2-hydroxybenzoyl]benzoate d'hexyle (INCI : Diethylamino hydroxybenzoyl hexyl benzoate), 2,4,6-tris-[anilino-(p-carbo-2'-éthyl-1'-hexyloxy)]-1,3,5-triazine (INCI : Ethylhexyl Triazone), 2,4-bis-{[4-(2-éthyl-hexyloxy)-2-hydroxy]-phényl}-6-(4-méthoxyphényl)-1,3,5-triazine (INCI : Bis-Ethylhexyloxyphenol methoxyphenyl Triazine).

14. Émulsion, utilisation ou procédé selon l'une des revendications précédentes, caractérisé(e) en ce qu'on utilise, comme émulsifiant, un ester d'acide gras de polyglycéryle-10.

15. Émulsion, utilisation ou procédé selon l'une des revendications précédentes, caractérisé(e) en ce qu'on utilise, comme émulsifiant, un ester d'acide gras de polyglycéryle-10 en une concentration de 0,1 à 2,0% en poids, par rapport au poids total de la préparation.

16. Émulsion, utilisation ou procédé selon l'une des revendications précédentes, caractérisé(e) en ce qu'on utilise comme émulsifiant du stéarate de polyglycéryle-10 (INCI : Polyglyceryl-10 Stearate) et/ou de l'oléate de polyglycéryle-10 (INCI : Polyglyceryl-10 Oleate).

17. Émulsion, utilisation ou procédé selon l'une des revendications précédentes, caractérisé(e) en ce qu'on utilise, comme émulsifiant, de la lécithine de soja en une concentration de 0,1 à 2,0% en poids, par rapport au poids total de la préparation.

18. Émulsion, utilisation ou procédé selon l'une des revendications précédentes, caractérisé(e) en ce que la préparation est exempte de 3-(4-méthylbenzylidène)-camphre, de 2-hydroxy-4-méthoxybenzophénone (INCI : Oxybenzone), d'ester 2-éthylhexylique de l'acide 4-méthoxycinnamique (INCI : Octylmethoxycinnamate), de 2-cyano-3,3-diphénylacrylate de 2-éthylhexyle, de salicylate d'homomenthyle, de parabènes (en particulier de propylparabène et de butylparabène), de méthylisothiazolinone, de chlorométhylisothiazolinone et d'hydantoïne DMDM, de polyéthylèneglycoléthers ou d'esters de polyéthylèneglycol ainsi qu'exempte d'huiles minérales, d'huiles de silicone et de cires minérales.

19. Émulsion, utilisation ou procédé selon l'une des revendications précédentes, caractérisé(e) en ce que la préparation contient un ou plusieurs composés choisis dans le groupe des composés acide alpha-liponique, acide folique, phytoène, D-biotine, coenzyme Q10, alpha-glucosylrutine, carnitine, carnosine, isoflavonoïdes naturels et/ou synthétiques, flavonoïdes, créatine, créatinine, taurine, β-alanine, panthénol, magnolol, honokiol, tocophérol, acétate de tocophéryle, dihydroxyacétone ; polydocanol, thiamidol, glycérylglucose, acide hyaluronique et/ou ses sels et/ou licochalcone A.
